# EUROPEAN PATENT APPLICATION

(11) **EP 1 709 985 A1**
(43) Date of publication of application: **11.10.2006**
(21) Application number: 05252167.1
(22) Date of filing: 07.04.2005
(51) Int. Cl.: A61M 5/32

(54) **Mechanism for tilting retractable needle**

(71) Applicant: Intai Technology Corp., Taichung City 407 (TW)
(72) Inventor: Lou, Jin-Rong, Taichung City 407 (TW)
(74) Representative: Brown, Michael Stanley

(57) **Abstract**

The present invention discloses a mechanism for tilting a retractable needle, in which a needle adapter (30) housed within a barrel (10) comprises a connecting tube (34) with a receiving groove (66) on inner wall. The connecting tube forms a rectangle hole (68) on the wall, and a cantilever (72) with at least one tongue (76) is formed between the rectangle hole and the rear portion of the connecting tube. The plunger fitted in the barrel comprises an extruding ring (62) at a front end thereof. When the plunger is pushed forwardly into the connecting tube of the needle-adapter, an exterior ring (22) of the plunger connected to with a piston will force the tongue to bend the cantilever toward the center of the open area, and engage the extruding ring into the receiving groove. When the plunger is pulled backward, the needle-adapter and the needle will be retracted into the barrel. Meanwhile, the needle will tilt as the tongue is forced against exterior ring by the cantilever, and the needle point is therefore close to the inner wall of the barrel. Accordingly, exposure of the needle point due to carelessly pushing the plunger can be prevented.

## Description

### BACKGROUND OF THE INVENTION

### 1. Field of the Invention

The present invention relates to a retractable mechanism and particularly, to a mechanism applied to a retractable safety syringe and capable of tilting the needle during retracting, so that injury by the needle point can be avoided.

### 2. Description of Related Prior Arts

In general, a syringe is composed of a hollow barrel a plunger. The barrel is associated with a needle and contains liquid. One end of the plunger is disposed with a piston for fitting in the barrel, and the other end is exposed from the barrel. By pushing the plunger, the liquid contained in the barrel will be injected from the needle point.

For the conventional syringe, the needle is necessary to be covered by a sheath before separated from the barrel. However, such behavior has a lot of risk of stabbing for the operator.

Taiwan Patent No. 394027 therefore provides a safety syringe, in which a needle with a support is housed in a barrel. The bottom of the support comprises two non-coplanar stopping elements. The center of two stopping elements is a cavity with a hook on the inner wall thereof. The plunger comprises a connector at its piston end, so that the connector can be engaged with the hook aforementioned when pushing the plunger forwardly. Accordingly, when the plunger is pulled rearward, the needle can be retracted back into the barrel and tilt due to the non-coplanar stopping elements and against to the piston.

As the wall of the needle-adapter is continuous and the cavity has a hook-shaped inner edge, the wall could not be resilient or bent. Therefore, when the connector enters into the cavity and engages to the hook, the non-coplanar stopping elements will block the connector. As a result, engagement between the hook and the connector becomes impossible. To solve this problem, the connector must comprise a resilient end surface as the piston, so that the stopping elements are possibly forced into the deformable connector.

However, the pistons of all commercial syringes have unresilient end surfaces made from rigid plastic material. That is, the above retractable mechanism is infeasible.

### SUMMARY OF THE INVENTION

An object of the present invention is to provide a mechanism for tilting a retractable needle, which enable the needle to tilt during retracting the needle back into the barrel.

Another object of the present invention is to provide a mechanism for tilting a retractable needle, which is suitable for pistons whether have resilient material on the front ends or not, and comprises a plunger and a needle with function of engagement.

To achieve the above objects, a needle-adapter with a receiving groove, a rectangle hole and a cantilever with a tongue is disposed in a barrel of the syringe; and a plunger with an extruding ring at a front end thereof is inserted in the barrel. When the plunger is pushed into a connecting tube of the needle-adapter, an exterior ring connected to a piston of the plunger will force a tongue of a cantilever to bend toward a center of the open area, and the extruding ring will engage into the receiving groove. When the plunger is pulled rearward, the needle will be retracted back into the barrel with needle adapter and tilt to the inner wall of the barrel by resilience of the cantilever and forcing the tongue against the exterior ring.

### BRIEF DESCRIPTION OF THE DRAWINGS

Fig. 1 is an explosive view of the first embodiment in accordance with the present invention.
Fig. 2 is a combinative view of the first embodiment.
Fig. 3 shows the needle adapter of the first embodiment.
Figs. 4 and 5 are cross sections of the syringe of the first embodiment in use.
Fig. 6 shows the needle and needle adapter retracted into the barrel according to the first embodiment.
Fig. 7 is an explosive view of the second embodiment in accordance with the present invention.
Fig. 8 is a combinative view of the second embodiment.
Fig. 9 shows the needle-adapter of the second embodiment.
Fig. 10 shows the needle-adapter of the third embodiment.
Figs.11 and 12 are cross sections of the syringe of the second embodiment in use.
Fig. 13 shows the needle and needle adapter retracted into the barrel according to the second embodiment.

### DETAILED DESCRIPTION OF THE PREFERRED EMBODIMENTS

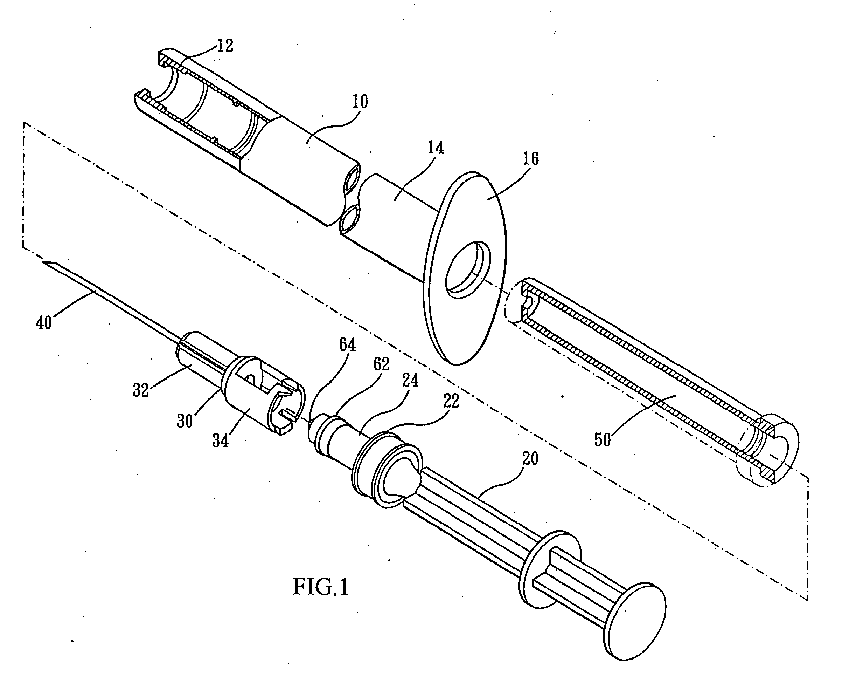

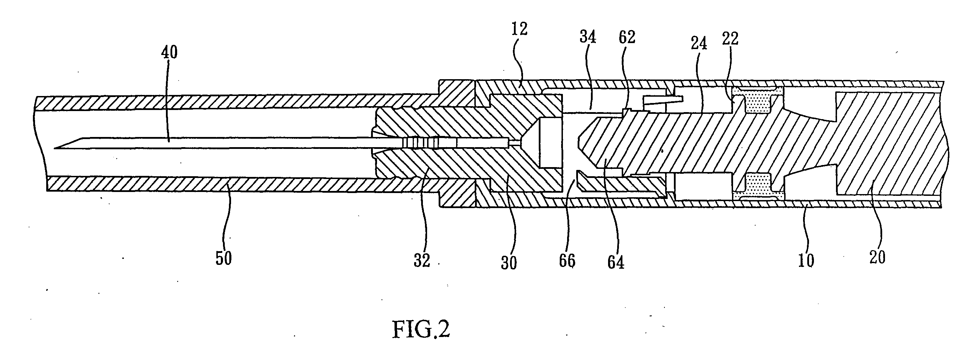

Figs. 1 and 2 are respectively explosive and combinative views of the first embodiment in accordance with the present invention. A syringe comprises a barrel 10, a plunger 20, a needle adapter 30, a needle 40 disposed at one end of the needle adapter 30, and a sheath 50 used to cover the needle 40.

The barrel 10 is a hollow tube having a stepped cross section at its front lumen 12 and an outer extension 16 at its rear lumen 14.

The needle- adapter 30 is arranged in the front lumen 12 of the barrel 10 and stopped by the stepped inner wall. One end of the plunger 20 comprises an exterior ring 22 connected with a piston and inserted in the barrel 10. By pushing the plunger 20, the exterior ring 22 will move toward the needle- adapter 30.

The exterior ring 22 of the plunger 20 further comprises a shaft 24 at its front end. The fore part of the shaft 24 is surrounded with an extruding ring 62 and a protrusion 64 is formed at the front end thereof.

The needle- adapter 30 comprises a tip 32 associated with the needle 40 and a hollow connecting tube 34 connected with the tip 32. The tip 32, the connecting tube 34 and the needle 40 are fluid communicable.

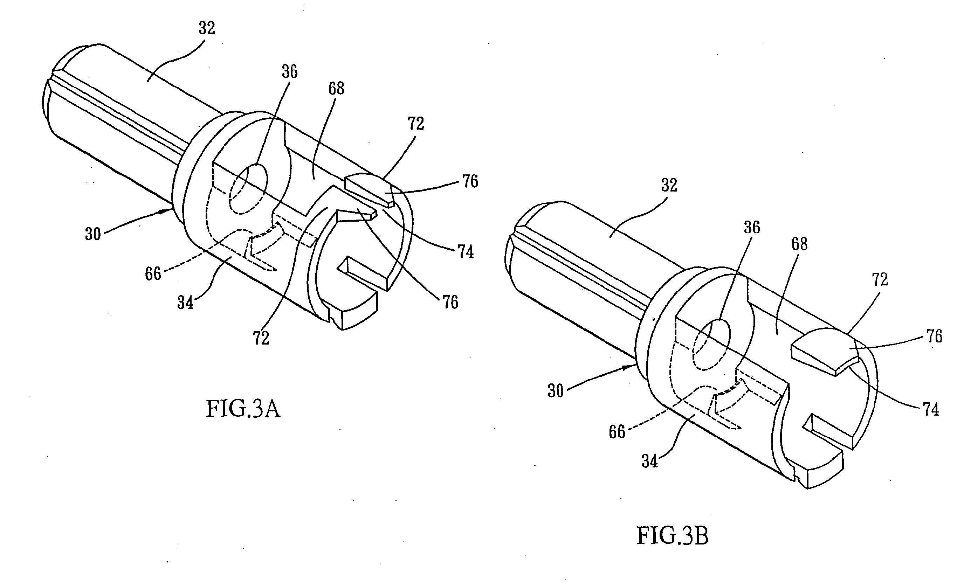

Fig. 3 shows the needle adapter of the first embodiment. The tip 32 of the needle-adapter 30 comprises an aperture 36 at rear end thereof. A receiving groove 66 is formed on the inner wall of the connecting tube 34 with a rectangle hole 68 on the wall of the connecting tube 34. In Fig. 3A, two cantilevers 72 are formed at one end of the rectangle hole 68 and separate by a space 74. Each of the cantilevers 72 comprises a tongue 76 at a free end thereof. The cantilevers 72 extend from the rear part of the connecting tube 34 and occupy a part of the rectangle hole 68. The tongues 76 also rearward extend a distance from the rear edge of the connecting tube 34.

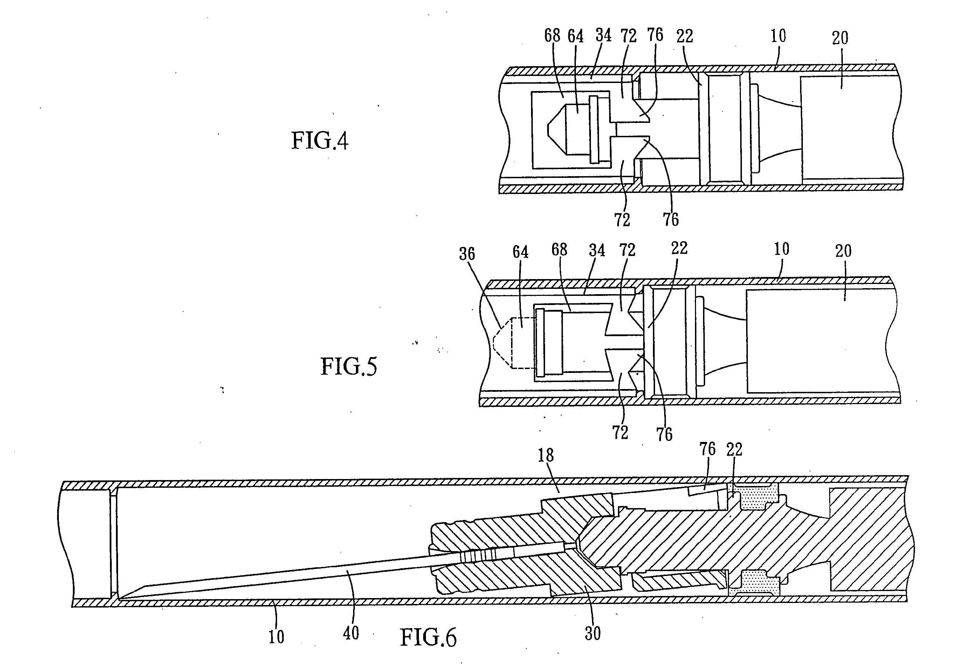

Figs. 4 and 5 are cross sections of the syringe of the first embodiment in use, in which the shaft 24 of the plunger 20 gradually approaches the connecting tube 34. Eventually, the protrusion 64 enters into the aperture 36, and the extruding ring 62 engages into the receiving groove 66. Meanwhile, the cantilever 72 is forced by the exterior ring 22 and bended toward the center of the rectangle hole 68.

After an injection is completed, the plunger 20 will be pulled rearward. The needle- adapter 30 is also retracted back into the middle 18 of the barrel 10 with the plunger 20 because of engagement between the extruding ring 62 and the receiving groove 66. Particularly, the middle 18 of the barrel 10 with a larger inner diameter has enough space for the needle-adapter 30 to tilt therein. Fig. 6 shows the needle 40 and needle adapter 30 retracted into the barrel 10.

As shown in Fig. 6, the cantilevers 72 tilt as the tongues 76 of the needle- adapter 30 is forced by the exterior ring 22 of the plunger 20. Therefore, when the needle- adapter 30 is retracted into the middle 18 of the barrel 10 with the plunger 20, the needle- adapter 30 can tilt in the middle 18 of the barrel 10 due to a fulcrum formed by engagement between the extruding ring 62 of the plunger 20 and the receiving groove 66 and axial resilience provided by the cantilever 72.

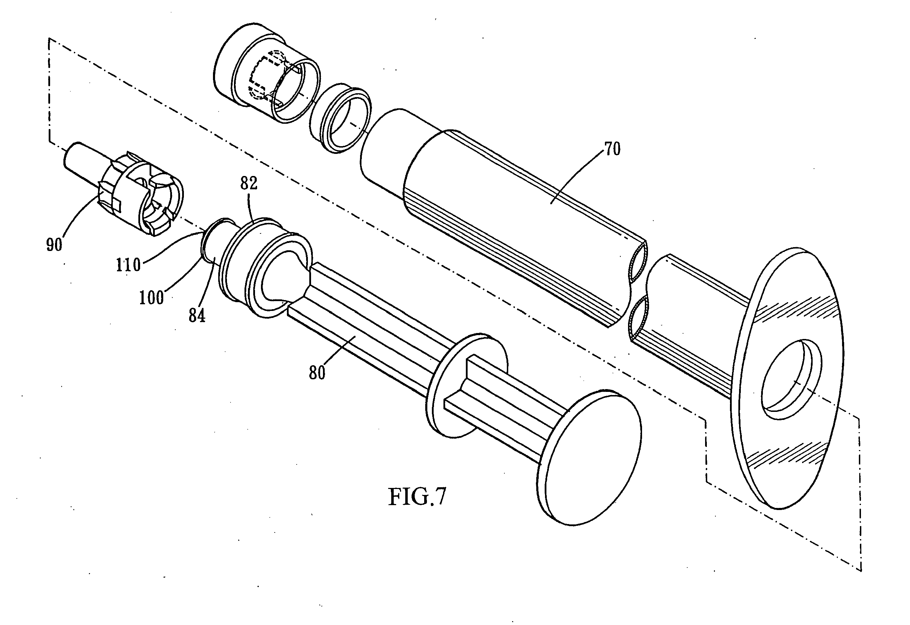

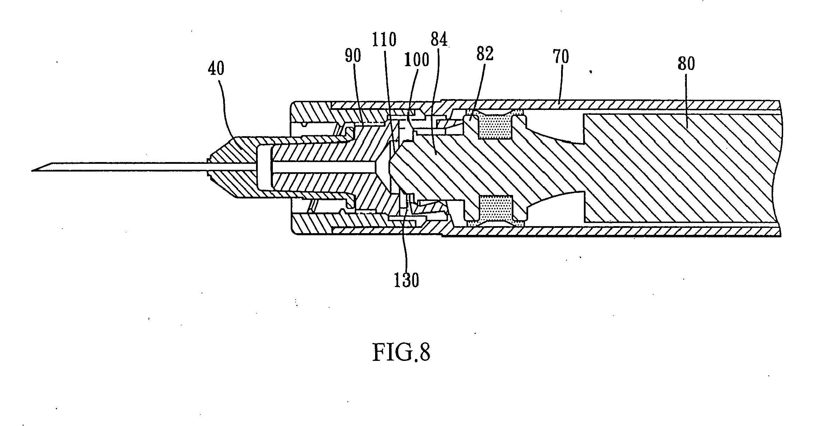

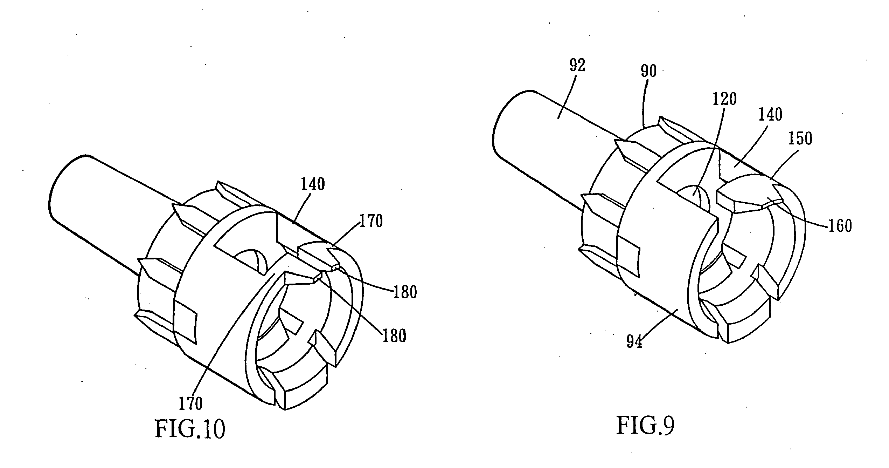

Figs. 7 and 8 are explosive and combinative views of the second embodiment in accordance with the present invention. A syringe comprises a barrel 70, a plunger 80 and a needle-adapter 90. The barrel 70 is similar to the first embodiment, and the exterior ring 82 of the plunger 80 connected with a shaft 84 at its front end. The shaft 84 is surrounded with an extruding ring 100 at its fore part and has a protrusion 110 at its front end. The plunger 80 is inserted from the rear end of the barrel 70, and the needle-adapter 90 associated with a needle 40 is disposed in the front end of the barrel 70. Fig. 9 shows the needle-adapter of the second embodiment. The needle-adapter 90 comprises a tip 92 at its front end, and a connecting tube 94 at its rear end. An aperture 120 is formed at the rear end of the tip 92. The connecting tube 94 comprises a receiving groove 130 on its inner surface. A rectangle hole 140 is formed on a wall of the connecting tube 94. A cantilever 150 extends from the rear part of the connecting tube 94 and occupies a part of the rectangle hole 140. The cantilever 150 has a tongue 160 at its free end, and the tongue 160 rearward extends a distance from the rear edge of the connecting tube 94.

Fig. 10 shows the needle-adapter of the third embodiment, which is similar to that shown in Fig. 9. Difference between them is that two cantilever 170 with tongues 180 at respective free end thereof are disposed in one end of the rectangle hole 140.

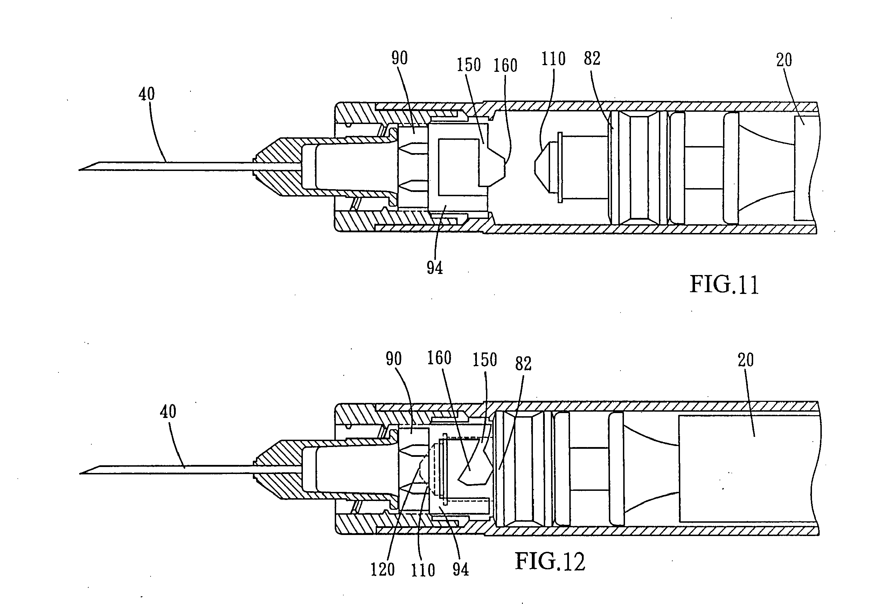

Figs.11 and 12 are cross sections of the syringe of the second embodiment in use. During injection, the plunger 20 is pushed into the connecting tube 94 of the needle-adapter 90. The protrusion 110 is then fitted into the aperture 120, and the extruding ring 100 is engaged into the receiving groove 130. Meanwhile, the tongue 160 is forced by the ring 82, so that the cantilever 150 can bend toward the center of the rectangle hole 140.

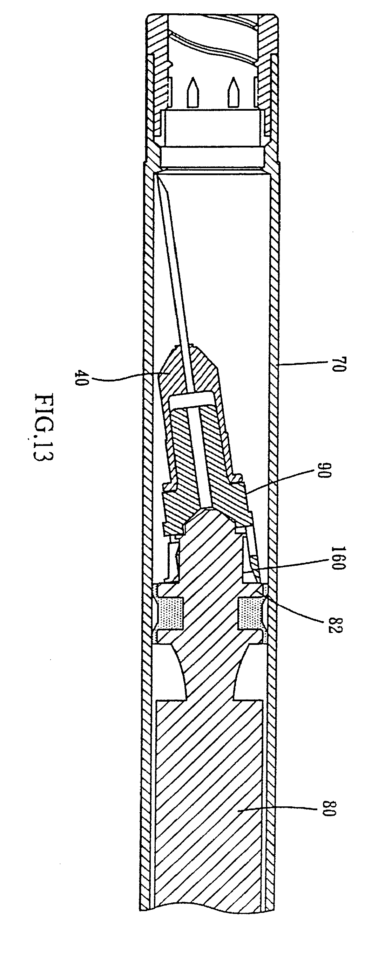

Fig. 13 shows the needle and needle adapter retracted into the barrel according to the second embodiment. When the plunger 80 is pulled rearward, the needle-adapter 90 is retracted back into the barrel 70 with the plunger 80 because of engagement between the extruding ring 82 and the receiving groove 130. Particularly, the barrel 70 with a larger inner diameter has enough space for the needle-adapter 90 to tilt therein. The cantilever 150 tilts as the tongue 160 of the needle-adapter 90 is forced by the exterior ring 82 of the plunger 80. Therefore, when the needle-adapter 90 is retracted into the barrel 70 with the plunger 80, the needle-adapter 90 can tilt in barrel 70 due to a fulcrum formed by engagement between the extruding ring 100 of the plunger 80 and the receiving groove 130 and axial resilience provided by the cantilever 150.

According to the above preferred embodiments, the needle-adapter of the present invention primarily comprises a rectangle hole, at least one cantilever with a tongue. By resilience of the cantilever and pushing force of the tongue, the needle will tilt toward the inner wall of the barrel. Such design can further prevent the needle point from stabbing. Moreover, as the cantilever of the present invention is deformable, any plunger with or without a resilient piston end can be engaged with the needle-adapter.

While the preferred embodiments of the present invention have been described in reference to the environment in which they were developed, they are merely illustrative of the principles of the inventions. Other embodiments and configurations may be devised without departing from the spirit of the inventions and the scope of the appended claims.

## Claims

1. A mechanism for tilting a retractable needle, which is arranged with a needle adapter and a plunger in a barrel, wherein said needle-adapter comprises a smaller end associated with a needle, and a larger end defining a hollow connecting tube, and said plunger comprises an extruding ring at a front end thereof, and said mechanism comprising:
a rectangle hole positioned at the circumference of said connecting tube;
at least one cantilever disposed on (an) edge(s) of said rectangle hole and having a free end and a fix end;
at least one tongue disposed on the free end of said cantilever;
a receiving groove disposed on an inner wall of said connecting tube;
an extruding ring disposed on an front end of said plunger;
accordingly, when said front end of said plunger enters into said connecting tube, an exterior ring disposed at middle of said plunger makes said extruding ring slide into said receiving groove for engagement with each other, and said cantilever bended toward center of said rectangle hole by pushing said tongue of said cantilever; so that said needle and needle adapter thereof will be retracted back into said barrel when pulling said plunger backward due to the engagement of said extruding ring of said plunger and said receiving groove of said needle-adapter, and particularly, said needle will tilt as axially pushed by said cantilever with a fulcrum formed by said extruding ring of said plunger and said receiving groove.

2. The mechanism as claimed in claim 1, wherein said connecting tube of said needle adapter comprises a receiving groove on one side of a wall thereof, and thus is engaged with an extruding ring at a front end of said plunger.

3. The mechanism as claimed in claim 1, wherein said receiving groove formed on said connecting tube of said needle adapter is disposed opposite to said rectangle hole, and one of said cantilever has a fix end connects to a wall of said connecting tube and a free end separate from a space of said connecting tube.

4. The mechanism as claimed in claim 1, wherein said receiving groove formed on said connecting tube of said needle adapter is disposed opposite to said rectangle hole, and two cantilevers are disposed oppositely on one side of said rectangle hole, and each of said cantilever has a fix end connects to a wall of said connecting tube and a free end separate from each other.

5. The mechanism as claimed in claim 1, wherein said tongue of said cantilever outwardly axially extends a distance from said free end of said cantilever.
